# FASCICULE DE BREVET EUROPEEN

(11) **EP 2 839 859 B1**
(45) Date de publication et mention de la délivrance du brevet: **27.04.2016**
(21) Numéro de dépôt: 14170624.2
(22) Date de dépôt: 30.05.2014
(51) Int. Cl.: A61N 1/362, A61N 1/37, A61B 5/0215, A61B 5/0452, A61B 5/11, A61N 1/05, A61B 5/08, A61B 5/00

(54) **Dispositif médical actif, notamment resynchroniseur CRT, comprenant des moyens d'alerte prédictive de décompensation cardiaque en présence d'apnées centrales du sommeil**
Aktive medizinische Vorrichtung, insbesondere Einheit für kardiale Resynchronisationstherapie (CRT), die Alarmmittel zur Vorhersage einer kardialen Dekompensation im Fall einer zentralen Schlafapnoe umfasst
Active medical device, in particular a CRT resynchroniser, including predictive warning means for cardiac decompensation in the presence of central sleep apnoea

(30) Priorité: 20.08.2013 FR 1358092
(43) Date de publication de la demande: 25.02.2015
(73) Titulaire: Sorin CRM SAS, 92140 Clamart Cedex (FR)
(72) Inventeur: Milpied, Paola, 75014 Paris (FR)
(74) Mandataire: Dupuis-Latour, Dominique

(56) Documents cités:
- EP-A1- 1 413 330
- EP-A1- 1 433 496
- EP-A1- 1 741 386
- EP-A1- 1 741 387

## Description

L'invention concerne les "dispositifs médicaux implantables actifs" tels que définis par la directive 90/385/CEE du 20 juin 1990 du Conseil des communautés européennes, plus particulièrement les dispositifs de stimulation cardiaque, resynchronisation et/ou défibrillation destinés aux diagnostics et au traitement des troubles du rythme cardiaque, y compris les implants actifs à visée purement diagnostique.

Elle concerne en particulier le diagnostic préventif du risque de décompensation cardiaque au moyen d'algorithmes d'analyse de signaux recueillis par le dispositif implanté, diagnostic qui peut être particulièrement utile avec les implants mettant en oeuvre des fonctions de resynchronisation.

La resynchronisation est une technique, dite "CRT" (*Cardiac Resynchronisation Therapy*) ou "BVP" (*Bi-Ventricular Pacing*), consistant à délivrer une stimulation conjointe et permanente aux ventricules gauche et droit, avec application éventuelle entre les instants respectifs de stimulation d'un délai, dit délai interventriculaire ou DVV. Ce délai est ajusté de manière à resynchroniser la contraction des deux ventricules, ce qui améliore l'état hémodynamique du patient en optimisant le cycle contraction/relaxation avec un bénéfice direct pour le coeur, dont le travail est facilité.

Les effets sur le long terme d'une thérapie CRT sur des patients insuffisants cardiaques peuvent être toutefois très différents. Ces effets sont notamment évalués par détermination de la fraction d'éjection gauche et de la classe NYHA d'insuffisance cardiaque, et comparaison des données initiales avec celles réévaluées par exemple au bout de six mois de traitement par thérapie CRT.

Si l'on constate chez la majorité des patients une amélioration de l'état clinique (qui se traduit par une augmentation de la fraction d'éjection gauche), chez d'autres patients on ne constate pas d'amélioration notable, et chez d'autres patients encore on constate bien au contraire une aggravation de l'état clinique, avec diminution de la fraction d'éjection gauche, voire avec augmentation d'une classe NYHA.

Le point de départ de l'invention est la constatation que la plupart de ces patients non répondeurs à la thérapie CRT souffrent d'apnées du sommeil, plus précisément d'apnées centrales (non obstructives). Les apnées centrales, qui ont une cause neurologique et ne résultent pas d'un obstacle à l'inspiration, peuvent en effet apparaitre comme une conséquence de l'insuffisance cardiaque.

De ce fait, la mise en place d'alertes concernant les risques de décompensation par les patients insuffisants cardiaques souffrant d'apnée du sommeil permettrait de modifier et d'adapter sans délai les traitements, et de prévenir les hospitalisations.

Il a déjà été proposé diverses techniques de détection des apnées du sommeil et d'application de thérapies en réponse.

Notamment, le EP 1 433 496 A1 (ELA Médical) décrit un dispositif pourvu de moyens d'évaluation du paramètre dit HRV (*Heart Rate Variability,* variabilité du rythme cardiaque) afin de caractériser la survenue d'épisodes d'apnée ou d'hypopnée, ceci exclusivement par analyse de la variabilité du segment R-R. Par ailleurs, ce document n'aborde en aucune façon la question des patients insuffisants cardiaques.

Ainsi, le EP 1 413 330 A1 (ELA Médical) propose de monitorer le signal de ventilation-minute (signal MV) pour détecter les épisodes d'apnée ou d'hypopnée et, simultanément, évaluer la contractilité du myocarde, par exemple par analyse d'un signal d'impédance transthoracique ou d'un signal d'accélération endocardiaque. En cas de variation significative de la contractilité lors d'un épisode d'apnée ou d'hypopnée, le dispositif modifie temporairement un paramètre de stimulation, par exemple la fréquence de stimulation ou le délai atrioventriculaire dans le cas d'un dispositif double chambre. Cette action permet de compenser par une augmentation de débit la désaturation en oxygène résultant de l'activité insuffisance du système sympathique pendant les épisodes de trouble respiratoire. L'objet de ce dispositif connu est de déclencher une réaction particulière du dispositif pendant les épisodes d'apnée seulement si cette réaction est appropriée ; il ne s'agit pas de monitorer sur le long terme l'état clinique d'un patient insuffisant cardiaque ni de déclencher des alertes en cas d'aggravation avérée de cet état clinique sur le long terme.

Le EP 1 741 386 A1 (ELA Médical) décrit un dispositif comparable, dans lequel la survenue d'épisodes d'apnée est détectée non plus par analyse d'un signal MV ou d'impédance transthoracique, mais par celle d'un signal d'accélération endocardiaque (EA). Une dépression soudaine de la contractilité cardiaque, associée à la survenue d'un épisode d'apnée ou d'hypopnée, active une alerte qui peut être utilisée pour déclencher une thérapie appropriée (augmentation de la fréquence de stimulation) et/ou inscrire des informations de diagnostic dans une mémoire du dispositif : marqueur de survenue d'une apnée, durée de l'alerte, etc.

Le EP 1 741 387 A1 (ELA Médical) décrit un dispositif de même nature, opérant à partir d'un dispositif externe comprenant un capteur cardiophonographique plaqué contre la paroi thoracique ainsi qu'un enregistreur EEG, donc applicable à des patients non appareillés d'un implant cardiaque. En tout état de cause, ce document a pour objet de détecter des apnées ou hypopnées à un instant donné sur la base de l'analyse d'un ratio moyenne à long terme/moyenne à court terme ; il ne s'agit en aucune façon d'évaluer un état général du patient nuit après nuit dans le cadre d'un diagnostic spécifique de l'insuffisance cardiaque.

Le EP 1 867 360 A2 (ELA Médical) propose de combiner entre eux divers signaux issus d'un capteur d'activité (capteur G, accélérométrique), d'un capteur physiologique (capteur MV, de ventilation-minute) et d'accélération endocardiaque (capteur EA). Les algorithmes d'analyses respectives produisent chacun un signal d'alerte de décompensation cardiaque et des moyens d'analyse croisée délivrent un signal composite d'alerte préventive, sur différents niveaux, en fonction d'un certain nombre d'index spécifiques susceptibles de révéler une aggravation de l'état clinique du patient. Toutefois, cette manière de procéder n'a pas pour objet d'évaluer l'efficacité d'une thérapie CRT, et ne présente pas de sensibilité ni de spécificité particulière à l'égard des patients non-répondeurs à une thérapie CRT, qui constitue le problème principal que cherche à résoudre la présente invention. En effet, outre le fait que cette technique connue ne cherche pas à détecter la survenue éventuelle d'épisodes d'apnée du sommeil, l'état clinique globalement évalué reste sensible à des facteurs tels que le niveau général d'activité du patient, le but étant de détecter et prendre en compte les réductions éventuelles d'activité lorsque le patient se ménage après avoir subi des premières crises apparaissant à l'effort. Or ces facteurs ne sont pas significatifs lorsque la dégradation de l'état cardiaque est corrélée à des troubles d'apnée du sommeil ; ils peuvent même empêcher une détection précoce du risque de décompensation cardiaque notamment dans le cas où le patient maintient un niveau d'activité stable tant qu'il n'est pas confronté à une aggravation subite de son état.

Le but de l'invention est de remédier à ces difficultés et limitations, en proposant un dispositif permettant d'évaluer avec une sensibilité et une sélectivité élevées l'effet d'une pathologie respiratoire telle que l'apnée centrale du sommeil, de façon spécifique sur l'évolution de l'état clinique d'un patient insuffisant cardiaque.

Un autre but de l'invention est de proposer un tel dispositif qui soit particulièrement et spécifiquement adapté à la discrimination rapide des patients non-répondeurs parmi une population de patients appareillés avec un dispositif CRT, de manière à pouvoir diagnostiquer sans retard ces patients et éviter de faire perdurer une thérapie inefficace, voire délétère, et de réévaluer rapidement la thérapie appliquée de manière à éviter une dégradation de l'état général du patient et une possible hospitalisation de celui-ci.

Un autre but encore de l'invention est de proposer un tel dispositif qui ne nécessite pas de mesure directe d'un signal de respiration périodique, c'est-à-dire qui n'ait pas besoin de détecter spécifiquement la survenue de chaque épisode d'apnée ou d'hypopnée.

Le but de l'invention est en effet d'évaluer l'impact de ce trouble respiratoire sur l'état clinique d'un patient insuffisant cardiaque, il n'est pas de prendre une action compensatrice immédiate (augmentation de la fréquence cardiaque, etc.) en réponse à l'apnée lorsque celle-ci survient.

Ces buts sont résolus au moyen d'un dispositif comprenant, de manière en elle-même connue notamment d'après le EP 1 433 496 A1 précité : un capteur apte à délivrer un signal d'accélération endocardiaque, EA ; des moyens d'analyse de signal, aptes à extraire du signal EA un paramètre EA prédéterminé et à mémoriser ce paramètre EA sur les cycles cardiaques successifs ; des moyens de détermination d'une période de sommeil du patient ; et des moyens d'évaluation de l'état hémodynamique du patient, aptes à délivrer un indice d'état clinique du patient porteur du dispositif en fonction des variations du paramètre EA sur ladite période de sommeil du patient.

De façon caractéristique de l'invention, les moyens d'évaluation calculent un indice de variabilité du paramètre EA sur la période de sommeil, calculent un ratio entre cet indice de variabilité calculé et une valeur d'indice de variabilité de référence, et délivrent le ratio comme indice d'état clinique. Le dispositif comporte en outre des moyens de diagnostic de l'insuffisance cardiaque, aptes à délivrer un signal d'alerte d'aggravation de l'état du patient en réponse au franchissement par ledit ratio d'un seuil d'alerte prédéterminé.

L'indice de variabilité est déterminé à partir de l'écart-type des valeurs du paramètre EA sur la période de sommeil, de préférence l'écart-type pondéré par la moyenne de ces valeurs sur la même période.

Le paramètre EA peut être un paramètre du groupe comprenant : la valeur de l'amplitude crête-à-crête de la composante EA1 ; la valeur de l'amplitude crête-à-crête de la composante EA2 ; l'intervalle de temps séparant le début de la composante EA1 du début de la composante EA2 ; le temps d'apparition de la composante EA1, représenté par l'intervalle séparant i) un marqueur temporel de début de cycle cardiaque et ii) le franchissement d'un seuil d'amplitude ou d'énergie de la composante EA1 ; l'intervalle de temps séparant i) le franchissement du seuil d'amplitude ou d'énergie de la composante EA1 de ii) l'instant du pic de la composante EA1 ; et un paramètre composite combinant des paramètres précédents. La composante EA1 est la composante correspondant au premier pic du signal EA associé à la contraction ventriculaire isovolumique, et la composante EA2 est la composante correspondant au second pic du signal EA associé à la relaxation ventriculaire isovolumique.

Selon diverses caractéristiques subsidiaires avantageuses :
- le dispositif comprend une horloge interne et des moyens aptes à restreindre la détermination de la période de sommeil à une plage horaire quotidienne prédéterminée ;
- le dispositif comprend des moyens de détection de phases d'activité du patient, et des moyens pour exclure du calcul de l'indice de variabilité les valeurs du paramètre EA mémorisées pendant des phases d'activité détectées au cours de la période de sommeil ;
- le dispositif comprend des moyens de détection de phases de réveil du patient, et des moyens pour exclure du calcul de l'indice de variabilité les valeurs du paramètre EA mémorisées pendant des phases de réveil détectées au cours de la période de sommeil ;
- le dispositif comprend un compteur de franchissements par le ratio du seuil d'alerte prédéterminé, et les moyens de diagnostic ne délivrent le signal d'alerte que lorsque le compteur atteint une valeur cumulée prédéterminée ; de préférence, le compteur n'est incrémenté qu'en cas de franchissements consécutifs du seuil d'alerte prédéterminé.

La valeur d'indice de variabilité de référence peut être une valeur fixe prédéterminée ou, de préférence, une valeur de l'indice de variabilité initialement calculée, puis mémorisée par les moyens d'évaluation.

Le dispositif peut enfin comprendre des moyens pour, lorsque le ratio franchit un seuil prédéterminé d'amélioration d'état du patient inférieur au seuil d'alerte prédéterminé, mettre à jour, par la valeur d'indice de variabilité courante calculée, la valeur d'indice de variabilité de référence mémorisée par les moyens d'évaluation.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une série de trois chronogrammes illustrant différents signaux caractéristiques recueillis au cours d'un cycle cardiaque.
La Figure 2 montre de façon plus détaillée l'allure du signal d'accélération endocardiaque au cours d'un cycle donné.
La Figure 3 est un diagramme montrant la répartition de patients faisant l'objet d'une thérapie CRT entre patients répondeurs et patients non-répondeurs, en fonction du taux d'apnées centrales et du ratio de variabilité de l'amplitude du premier pic d'accélération endocardiaque.
Les Figures 4a et 4b sont des relevés des variations de l'amplitude du premier pic d'accélération endocardiaque au cours du temps pendant une période de sommeil d'un patient non-répondeur, respectivement au début de la thérapie et six mois après, montrant l'augmentation marquée de la variabilité de cette valeur d'amplitude au fil de la dégradation de l'état général du patient.
La Figure 5 illustre schématiquement les différents paramètres intervenant pour l'évaluation selon l'invention de l'état clinique du patient, ainsi que les phases successives de calcul d'un indice représentatif de cet état clinique.
La Figure 6 est un organigramme simplifié expliquant les différentes étapes à mettre en oeuvre pour évaluer l'état clinique du patient et déclencher si nécessaire une alerte, selon un premier mode de réalisation de l'invention.
La Figure 7 est semblable à la Figure 6, pour un second mode de réalisation de l'invention.

On va maintenant décrire un exemple de réalisation de l'invention. L'invention peut notamment être appliquée aux dispositifs implantables tels que ceux de la famille *Paradym* (en particulier le *Paradym RF SonR CRT-D*) produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts, mais cette représentation n'a toutefois qu'un caractère illustratif, ces blocs comprenant des éléments communs et correspondant en pratique à une pluralité de fonctions globalement exécutées par un même logiciel.

La technique de l'invention est basée sur l'analyse de l'accélération endo-cardiaque (ci-après désignée "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée dans l'oreillette ou le ventricule et intégrant un microaccéléromètre permettant de mesurer l'accélération endocardiaque.

Un tel capteur peut être par exemple disposé sur une sonde endocavitaire aboutissant au fond du ventricule et pourvue à cet endroit d'un capteur d'accélération. Il peut s'agir également d'une sonde auriculaire pourvue à son extrémité disposée contre la paroi de l'oreillette droite d'un capteur d'accélération. On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boitier d'un implant. L'invention est également applicable à l'analyse d'un signal EA externe recueilli de manière non invasive, par exemple issu d'un capteur fixé sur la poitrine du patient au niveau du sternum.

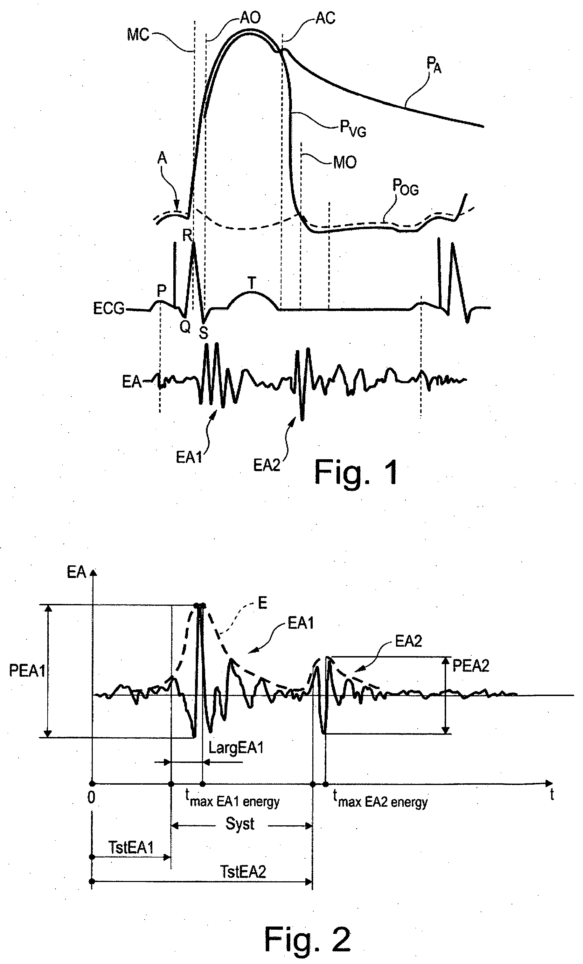

La Figure 1 illustre les différents signaux caractérisant l'activité du coeur au cours d'un cycle cardiaque, avec :
- le profil des pressions intracardiaques P_{A}, P_{VG} et P_{OG} : la caractéristique P_{A} illustre les variations de la pression aortique, P_{VG} celles du ventricule gauche et P_{OG} celles de l'oreillette gauche. Ces variations passent par les différentes phases suivantes : A contraction de l'oreillette gauche, MC fermeture de la valve mitrale, AO ouverture de la valve aortique, AC fermeture de la valve aortique, MO ouverture de la valve mitrale ;
- un relevé d'électrocardiogramme de surface ECG, avec successivement : l'onde P correspondant à la dépolarisation des oreillettes, le complexe QRS correspondant à la dépolarisation des ventricules, et l'onde T de repolarisation ventriculaire ; et
- les variations du signal EA d'accélération endocardiaque recueilli, qui forme deux composantes principales EA1 et EA2 au cours d'un cycle cardiaque donné, correspondant aux deux bruits majeurs du coeur (sons S1 et S2 du phonocardiogramme) qu'il est possible de reconnaitre dans chaque cycle cardiaque.

Sur la Figure 2, on a illustré plus précisément les variations de ce signal EA au cours d'un cycle cardiaque, montrant :
- la composante EA1, qui débute à la suite du complexe QRS et qui est engendrée par une combinaison de la fermeture des valves atrioventriculaires (valve mitrale et valve tricuspide), de l'ouverture des valves semi-lunaires (valve aortique et valve pulmonaire) et de la contraction du ventricule gauche. Les variations d'amplitude de cette composante EA1 sont étroitement liées aux variations de la pression dans le ventricule, l'amplitude maximale crête-à-crête PEA1 étant plus précisément corrélée au maximum positif de la variation de pression dP/dt dans le ventricule gauche ; et
- la composante EA2, qui survient pendant la phase de relaxation ventriculaire isovolumique. Elle accompagne la fin de la systole ventriculaire et est principalement produite par la fermeture des valves aortique et pulmonaire.

Pour la mise en oeuvre de l'invention, il convient d'extraire du signal EA un paramètre prédéterminé, ci-après "paramètre EA", qui sera utilisé pour caractériser la présence ou non d'apnées centrales du sommeil.

Ce paramètre EA est obtenu par un procédé connu tel que celui décrit dans le EP 2 092 885 A1 (ELA Medical), qui permet notamment d'éliminer les variations cycle à cycle en recalant dans le temps les composantes successives avant de les moyenner.

Essentiellement, le signal EA fait l'objet d'un traitement préliminaire consistant à individualiser les cycles cardiaques successifs dans le signal EA recueilli en continu, en déterminant des marqueurs de début de cycle permettant de séparer ces cycles et d'isoler une série de sous-signaux EA bornés dans le temps correspondant chacun à une durée d'un seul cycle cardiaque. Dans le cas d'un signal EA endocavitaire, les marqueurs temporels de début de cycle peuvent être fournis par l'implant lui-même ou par un dispositif externe lors de l'implantation, qui selon le mode de fonctionnement garde en mémoire les instants de la stimulation V, ou bien les instants de détection de l'onde R.

L'étape suivante consiste à isoler les composantes EA1 et EA2 dans chacun des sous-signaux bornés dans le temps correspondant à un cycle cardiaque. Chacune de ces composantes EA1 et EA2 sera représentée par un ensemble de valeurs successives décrivant la variation continue du signal EA dans une fenêtre temporelle donnée s'étendant autour du pic atteint par le signal EA (pic PEA1 ou pic PEA2), sur une fraction de la durée d'un cycle cardiaque. Concrètement, chaque composante sera constituée d'un sous-ensemble des échantillons du signal EA obtenus après numérisation de ce signal sur la durée du cycle cardiaque.

Chacune de ces composantes représente donc une fraction du signal EA sur la durée d'un cycle cardiaque, chaque cycle cardiaque comprenant une pluralité de composantes différentes qui se succéderont. De préférence, les composantes EA1 et EA2 du signal EA sont déterminées avec un moyennage sur plusieurs cycles, typiquement trois à cinq cycles, en appliquant une technique telle que celle du EP 2 092 885 A1 précité, technique qui permet notamment d'éliminer les variations cycle à cycle en recalant dans le temps les composantes successives avant de les moyenner. Essentiellement, cette technique consiste à effectuer un prétraitement du signal EA recueilli en continu, avec :
- découpage du signal EA en sous-signaux correspondant chacun à la durée d'un cycle cardiaque et repérés par un marqueur de début de cycle permettant de réaliser ce découpage ;
- segmentation de chacun de ces sous-signaux de manière à individualiser les composantes EA1 et EA2 dans une fenêtre temporelle donnée ;
- pour la composante courante EA1 ou EA2 ainsi isolée sur un cycle, recherche d'un pic d'intercorrélation par rapport aux composantes EA1 (ou EA2) des autres cycles recueillis ;
- calcul d'un décalage temporel correspondant ; et
- application du décalage temporel ainsi calculé à la composante courante, de manière à aligner celle-ci par rapport aux autres.

Les traitements d'analyse pourront être ensuite exécutés sur ces composantes EA1 et EA2 successives, avec élimination du biais de la variabilité cycle à cycle grâce à ce prétraitement.

Le paramètre EA prédéterminé, qui sera utilisé pour caractériser la présence ou non d'apnées centrales du sommeil, est de préférence l'amplitude PEA1 du premier pic d'accélération endocardiaque, c'est-à-dire la valeur maximale crête-à-crête séparant les deux extrema, positif et négatif, de la composante EA1 du signal d'accélération. C'est ce paramètre PEA1 qui sera utilisé dans la suite de la description comme paramètre caractéristique, mais ce choix n'est cependant aucunement limitatif.

De façon générale, il est possible d'utiliser comme paramètre EA caractéristique (cf. Figure 2) :
- l'amplitude crête-à-crête de la composante EA1, désignée *PEA1* ;
- de façon comparable, l'amplitude crête-à-crête de la composante EA2, désignée *PEA2* ;
- le temps d'apparition du début de la composante EA1, noté *TstEA1*, qui est une durée comptée à partir de l'origine des temps O sur la Figure 2 (instant de la stimulation V ou bien de détection de l'onde R, correspondant à un marqueur de début de cycle) ;
- un indicateur de la durée de la composante EA1, désigné *LargEA1,* qui est l'intervalle de temps séparant *TstEA1* de l'instant *t_{maxEA1energy}* du pic d'énergie de la composante EA1 ;
- l'intervalle de temps *Syst* séparant *TstEA1*, qui marque le début de la composante EA1, de *TstEA2,* qui marque le début de la composante EA2, cet intervalle correspondant à la durée de la systole (les temps *TstEA1* et *TstEA2* de début des composantes EA1 et EA2 peuvent être obtenus par exemple en seuillant une enveloppe d'énergie E obtenue en relevant au carré la valeur des échantillons de signal, puis en appliquant une fenêtre de lissage et un seuillage) ; et/ou
- un paramètre composite combinant avec une pondération appropriée une pluralité des paramètres précédents.

Le point de départ de l'invention réside dans la constatation du fait qu'au moins 50 % des patients insuffisants cardiaques présentent des apnées du sommeil.

Dans certains cas, il s'agit d'apnées obstructives, essentiellement associées à l'obésité, qui sont les plus courantes et sont produites par une obstruction des voies aériennes supérieures (nez, bouche, larynx, pharynx). L'autre type d'apnées du sommeil, à savoir les apnées dites centrales, a une cause neurologique, donc sans cause liée au système respiratoire.

Contrairement aux apnées obstructives, les apnées centrales peuvent apparaitre comme une conséquence de l'insuffisance cardiaque, de sorte qu'une augmentation avérée des apnées centrales chez l'insuffisant cardiaque indique une nécessité d'intensifier la thérapie liée à l'insuffisance cardiaque.

La mise en place d'alertes visant les risques de décompensation pour les patients insuffisants cardiaques suite à une augmentation des apnées du sommeil permettrait de modifier les traitements et thérapies, et de prévenir les hospitalisations.

Plus précisément, la proposition de l'invention repose sur la constatation, lors d'études cliniques, que l'instabilité du signal EA serait un marqueur de décompensation chez l'insuffisant cardiaque : en d'autres termes, plus l'état du patient se détériore, plus la variabilité du signal EA augmente.

Il s'agit d'évaluer cette variabilité seulement pendant les phases de sommeil, afin de disposer d'un indice d'état clinique non lié à l'activité du patient, et qui au contraire soit principalement lié au nombre d'apnées du sommeil - conduisant ainsi à des valeurs de sensibilité et de spécificité bien meilleures à l'égard de cette pathologie particulière.

La variabilité du signal EA - qui est le paramètre essentiel de la technique de l'invention - est à distinguer de la variabilité de la fréquence cardiaque ou HRV (*Heart Rate Variability*) qui est un paramètre différent, parfois utilisé pour évaluer l'évolution sur le long terme d'un patient insuffisant cardiaque. Plus précisément, la HRV reflète la capacité du coeur à s'adapter à des circonstances qui changent, de sorte qu'une stabilité ou une faible variabilité de la fréquence cardiaque est un signe de dégradation de l'état du patient. À l'opposé, dans le cas de l'invention, l'analyse est opérée pendant une période de sommeil, donc une période sans effort ni perturbation, où le rythme cardiaque est naturellement et normalement stable de sorte que la contractilité ne devrait pas avoir à s'adapter : ainsi, si l'on constate une augmentation de la variabilité du signal EA pendant les périodes de sommeil, ceci met en évidence un comportement chaotique du coeur, révélant une dégradation de l'état clinique du patient insuffisant cardiaque.

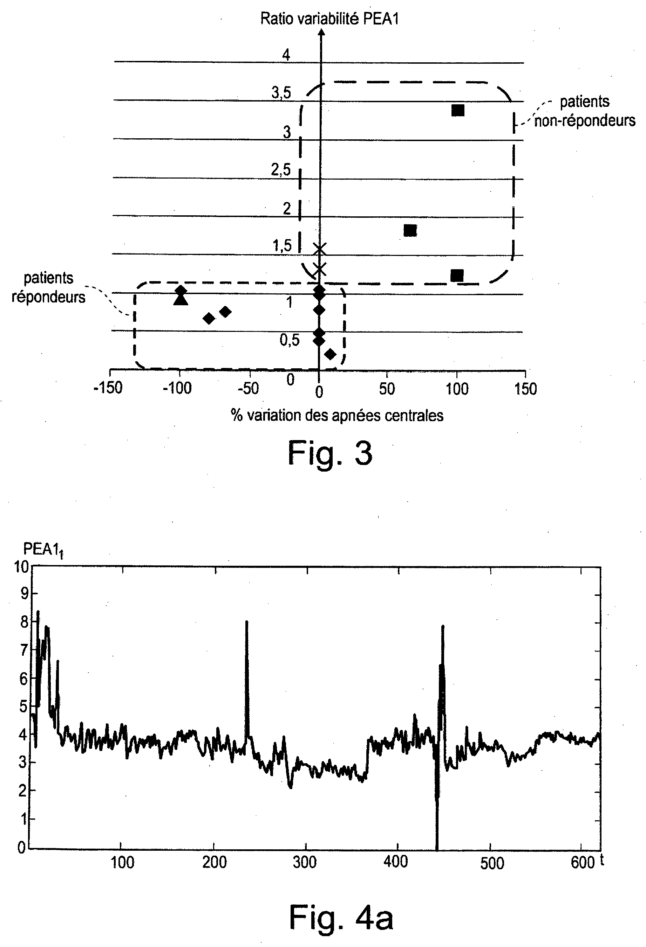

La Figure 3 illustre les résultats d'une étude clinique portant sur quinze patients appareillés d'un dispositif CRT. Ces patients ont fait l'objet d'une première analyse polysomnographique avant implantation du dispositif, puis d'une deuxième analyse six mois après. À chacun de ces examens, un signal EA a été enregistré, de manière à déterminer la variabilité (écart-type divisé par la valeur moyenne) du PEA1 pendant le sommeil et dans la position la plus fréquente, pour s'affranchir des modifications du signal EA liées à la position du patient (dans la mesure où, dans cette étude, les patients étaient appareillés avec un capteur externe ; dans le cas d'un capteur endocavitaire ce facteur serait sans incidence). Le ratio des variabilités du PEA1 entre les deux examens a été calculé et porté en ordonnée sur la Figure 3, avec en abscisse le taux de variation des apnées centrales.

On observe un premier groupe de patients, répondeurs à la thérapie CRT : ces patients présentent une amélioration d'au moins une classe NYHA (losanges sur la Figure 3), ou une augmentation significative de la fraction d'éjection gauche dans la même classe NYHA (triangle sur la Figure 3).

Un autre groupe de patients, en revanche, ne répondent pas à la thérapie CRT, et présentent une aggravation d'au moins une classe NYHA (carrés sur la Figure 3) ou ne présentent aucune augmentation significative de la fraction d'éjection gauche dans la même classe NYHA (croix sur la Figure 3).

On peut constater que pour les patients non-répondeurs le ratio de variabilité du PEA1 ne dépasse pas 1,1, tandis que pour les patients non-répondeurs ce ratio est supérieur à 1,2.

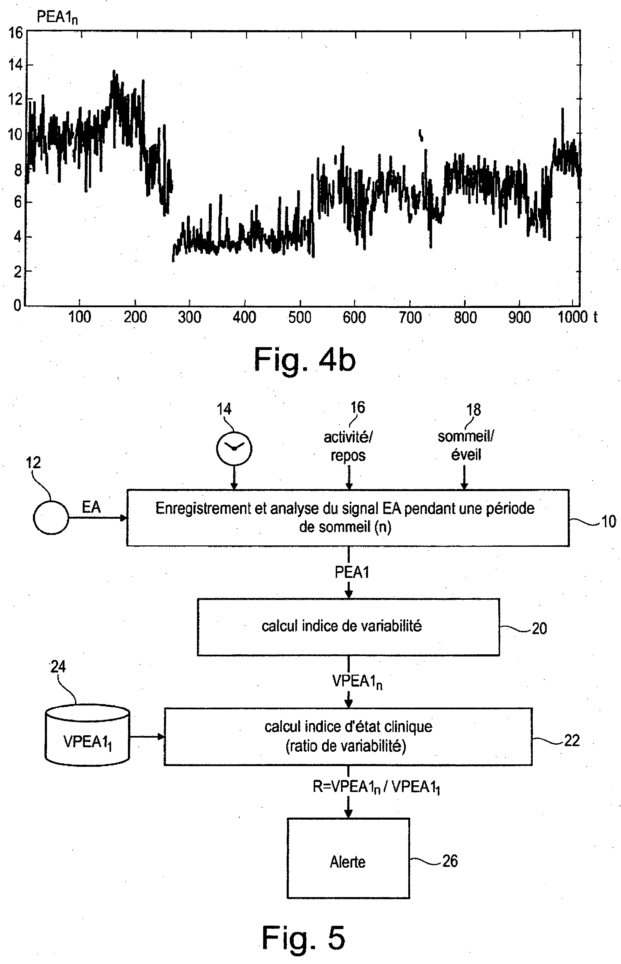

Les Figures 4a et 4b sont des relevés des variations de l'amplitude du premier pic d'accélération endocardiaque au cours du temps pendant une période de sommeil d'un patient non-répondeur, respectivement au début de la thérapie (Figure 4a) et six mois après (Figure 4b), montrant l'augmentation marquée de la variabilité de cette valeur d'amplitude au fil de la dégradation de l'état général du patient. Chez ce patient au bout de six mois d'application de la thérapie CRT, la classe NYHA s'est dégradée de III à IV, la fraction d'éjection gauche est passée de 23 à 12, et le nombre moyen d'apnées centrales par heure a augmenté de 22 à 45. Le ratio de variabilité du PEA entre les deux examens s'établit à 1,23.

L'idée de base de la présente invention est de créer une alerte lorsque le patient n'est pas répondeur à la thérapie CRT, en se basant sur la valeur du ratio susceptible de traduire l'augmentation ou la diminution des apnées centrales. Avec un seuil de 1,2, dans le cas des quinze patients décrits plus haut, la spécificité et la sensibilité seraient ainsi de 100 %.

On va maintenant décrire plus en détail la manière dont ce ratio caractéristique est évalué et dont l'alerte est déclenchée si nécessaire, en référence aux Figures 5 à 7.

La Figure 5 illustre schématiquement les différents paramètres intervenant à cet effet, ainsi que les phases successives de calcul d'un indice représentatif de l'état clinique du patient.

Le bloc 10 schématise les fonctions d'enregistrement des analyses du signal EA pendant une période de sommeil d'indice n : le signal EA est délivré par un capteur 12, interne ou externe, de préférence un capteur intégré à une sonde endocavitaire reliée à un générateur de dispositif CRT, une horloge interne 14 permet de définir des fenêtres temporelles prédéterminées, tandis qu'un capteur approprié, par exemple un capteur d'accélération (capteur G) délivre un signal 16 indicateur d'un état d'activité ou bien de repos du patient.

La discrimination 18 entre phases de sommeil et d'éveil peut être opérée par divers moyens.

La technique la plus simple consiste à utiliser l'horloge interne 14 du dispositif, commutant un indicateur à heures fixes. Il est également possible, comme enseigné par le EP-A-0 719 568 A1 (ELA Médical), d'opérer la discrimination entre éveil et sommeil par analyse du signal de ventilation-minute (MV) : en effet, la variation circadienne de la fréquence et de l'amplitude des cycles respiratoires successifs du patient est bien reproduite par ce signal, et un calcul de la ventilation moyenne sur 24 h permet d'opérer une discrimination satisfaisante entre une ventilation d'éveil et une ventilation de sommeil. On peut utiliser en complément le capteur d'activité 18, dont le signal permet de détecter les mouvements du patient : l'information de ce type de capteur n'est en elle-même pas très spécifique des phases d'éveil et de sommeil, mais on sait combiner les signaux délivrés par un capteur G et un capteur MV pour en déduire des informations signifiantes, comme cela est décrit par exemple dans les EP-A-0 750 920 A1, EP-A-0 770 407 A1 et EP 1 317 943 A1 (ELA Médical), auxquels on pourra se référer pour de plus amples détails.

Le bloc 10 évalue sur les cycles successifs de cette période de sommeil la valeur du paramètre EA représentatif choisi, qui dans cet exemple est l'amplitude *PEA1* du premier pic de la composante EA1 du signal EA.

La valeur *PEA1*est mesurée à chaque cycle cardiaque détecté, de préférence avec exclusion des composantes relevées pendant des phases éventuelles où un réveil et/ou une activité sont détectés pendant la période de sommeil considérée (toute la nuit). Une valeur moyenne est calculée sur une fenêtre mobile par exemple de 60 secondes, avec un recouvrement de 30 secondes entre les fenêtres successives, l'état sommeil/éveil étant donné toutes les 30 secondes. À partir de ces mesures successives, le dispositif évalue alors (bloc 20) un indice de variabilité *VPEA1ₙ* sur la nuit de rang n, cet indice étant calculé comme étant l'écart-type des valeurs *PEA1* successives sur la période n, divisé par la moyenne de ces valeurs *PEA1* sur toute la longueur de la nuit.

On notera que cette définition de la variabilité n'est pas limitative, et que d'autres indices de variabilité peuvent être envisagés, par exemple simplement à partir de l'écart-type des valeurs de *PEA1* non pondéré par la moyenne de ces valeurs sur la même période, ou encore l'écart-type, pondéré ou non, d'autres paramètres EA tels que ceux présentés en référence à la Figure 2.

Comme on l'a indiqué plus haut, dans la présente invention plutôt qu'à la valeur absolue de la variabilité *VPEA1ₙ* on préfère s'intéresser à l'évolution de cette variabilité, de manière à souligner un changement d'état sans juger l'état clinique à un moment donné.

Pour cela, on évalue (bloc 22) un ratio entre deux variabilités, à savoir la variabilité courante *VPEA1ₙ* et la variabilité initiale *VPEA1₁,* conservée dans une mémoire 24 du dispositif. Ce ratio *R* = *VPEA1ₙ*/*VPEA1* est comparé à un seuil prédéterminé : si la variabilité a augmenté au cours de la dernière nuit, on peut conclure à une aggravation de l'état clinique du patient au niveau du nombre d'apnées centrales et donc de l'insuffisance cardiaque, conduisant au déclenchement d'une alerte révélant un risque de décompensation cardiaque (bloc 26).

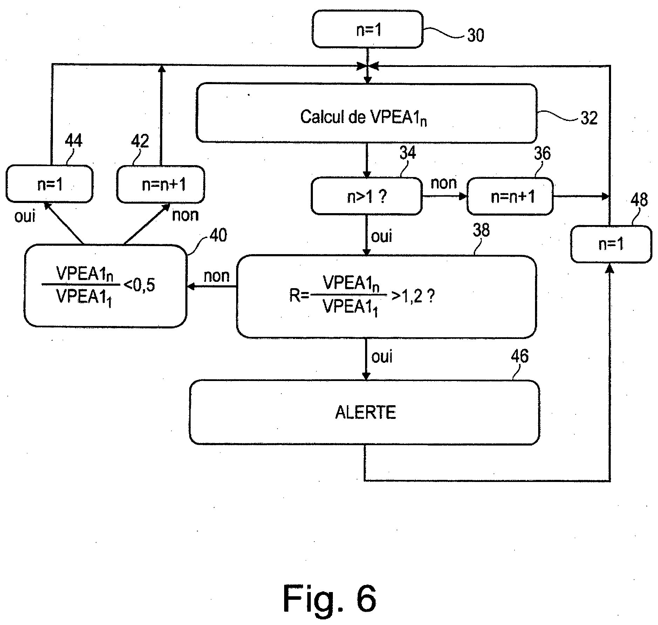

La Figure 6 illustre plus précisément l'algorithme de calcul du ratio de la variabilité et de déclenchement éventuel de l'alerte.

L'indice n, initialisé à *n* = 1 (bloc 30) indique le rang successif de la nuit au cours de laquelle l'état du patient est évalué. Après calcul de la variabilité *VPEA1ₙ* (bloc 32), s'il s'agit de la première nuit (test 34) la valeur de *VPEA1₁* est mémorisée et l'indice n est incrémenté d'une unité (bloc 36) avant retour au calcul de la valeur suivante de variabilité *VPEA1ₙ.*

Si le test 34 indique que la nuit n'est pas la première, alors le ratio *R* = *VPEA1ₙ*/ *VPEA1₁* est calculé et comparé au seuil prédéterminé, qui vaut par exemple 1,2 (bloc 38).

Si ce seuil n'est pas dépassé, le ratio est comparé à un autre seuil, notablement inférieur, par exemple un seuil de 0,5 (bloc 40) : si R est inférieur à cet autre seuil, ceci signifie que la variabilité est significativement plus faible lors de la dernière nuit et l'on peut donc conclure à une amélioration significative de l'état du patient. Il convient alors d'actualiser la valeur de référence *VPEA1₁* par remise à 1 de l'indice n (bloc 44). Dans le cas contraire, l'indice n est incrémenté d'une unité (bloc 42) avant retour au calcul de la variabilité *VPEA1ₙ* pour la nuit suivante (bloc 32).

Si, lors du test 38, le ratio R dépasse le seuil prédéterminé d'alerte, alors on peut conclure à une aggravation de l'état du patient, ce qui amène à déclencher l'alerte (bloc 46).

Si au cours des nuits successives le ratio ne dépasse jamais la valeur limite fixée (1,2 dans cet exemple), cela ne signifie pas forcément que le patient est répondeur à la thérapie CRT, mais seulement que le nombre d'apnées n'a pas augmenté significativement et que cela ne permet pas de conclure à une aggravation. D'autres paramètres peuvent être alors mesurés et évalués afin de détecter une aggravation de l'insuffisance cardiaque qui ne serait pas corrélée à une augmentation des apnées centrales, comme décrit par exemple dans le EP 1 867 360 A2 précité.

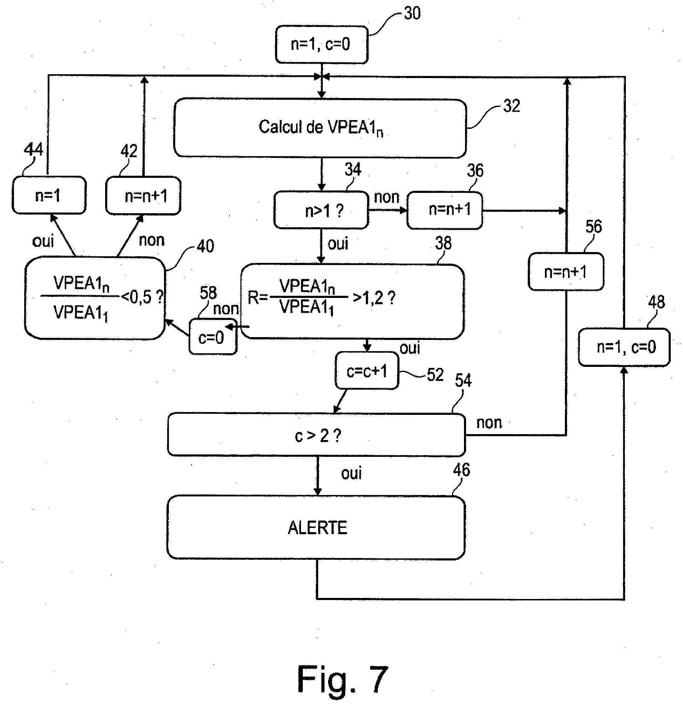

La Figure 7 illustre une variante de la Figure 6, qui prévoit de ne pas déclencher systématiquement l'alerte 46 dès que le seuil du test 38 est dépassé, mais seulement en cas de franchissements successifs, par exemple lorsque ce seuil est franchi au cours de deux nuits consécutives. Pour cela, l'algorithme (dont les blocs correspondant aux mêmes fonctions sont référencés identiquement à la Figure 6) inclut un compteur additionnel c de franchissements, initialisé à zéro au bloc 30. En cas de franchissement du seuil au test 38, ce compteur est incrémenté (bloc 52), et sa valeur est ensuite testée au bloc 54 : si c vaut 1, seul l'indice n est incrémenté (bloc 56) et le processus est réitéré. Dans le cas contraire, ceci signifie que le seuil a déjà été franchi au cours de la nuit précédente et il convient alors de déclencher l'alerte (bloc 46). Si le seuil a été franchi une première fois mais de façon non réitérée à la nuit suivante, alors le compteur c est remis à zéro (bloc 58) pour éviter une fausse alerte, ou bien dans les situations où le ratio est proche du seuil de déclenchement, nécessitant une confirmation de l'alerte la nuit suivante.

## Revendications

1. Un dispositif médical actif, notamment un dispositif implantable de stimulation, resynchronisation et/ou défibrillation, ou un appareil à visée diagnostique, comportant :
- un capteur (12) apte à délivrer un signal d'accélération endocardiaque, EA;
- des moyens (10) d'analyse de signal, aptes à extraire du signal EA un paramètre EA prédéterminé (*PEA1*) et à mémoriser ce paramètre EA sur les cycles cardiaques successifs ;
- des moyens (10) de détermination d'une période de sommeil (n) du patient ; et
- des moyens (20, 22) d'évaluation de l'état hémodynamique du patient, aptes à délivrer un indice d'état clinique du patient porteur du dispositif en fonction des variations du paramètre EA sur ladite période de sommeil (n) du patient,
**caractérisé en ce que** les moyens d'évaluation (20, 22) sont des moyens aptes à :
- calculer un indice de variabilité (*VPEA1ₙ*) dudit paramètre EA sur la période de sommeil (n),
- calculer un ratio (R) entre ledit indice de variabilité calculé et une valeur d'indice de variabilité de référence *(VPEA1₁),* et
- délivrer ledit ratio comme indice d'état clinique ;
et **en ce que** le dispositif comporte en outre des moyens (26) de diagnostic de l'insuffisance cardiaque, aptes à délivrer un signal d'alerte d'aggravation de l'état du patient en réponse au franchissement par ledit ratio d'un seuil d'alerte prédéterminé.

2. Le dispositif de la revendication 1, dans lequel ledit indice de variabilité est déterminé à partir de l'écart-type des valeurs du paramètre EA sur la période de sommeil.

3. Le dispositif de la revendication 2, dans lequel ledit indice de variabilité est l'écart-type des valeurs du paramètre EA sur la période de sommeil, pondéré par la moyenne de ces valeurs sur la même période.

4. Le dispositif de la revendication 1, dans lequel ledit paramètre EA est un paramètre du groupe comprenant :
- la valeur (*PEA1*) de l'amplitude crête-à-crête de la composante EA1 ;
- la valeur (*PEA2*) de l'amplitude crête-à-crête de la composante EA2 ;
- l'intervalle de temps (*Sys*t*)* séparant le début de la composante EA1 du début de la composante EA2 ;
- le temps (*TstEA1*) d'apparition de la composante EA1, représenté par l'intervalle séparant i) un marqueur temporel de début de cycle cardiaque et ii) le franchissement d'un seuil d'amplitude ou d'énergie de la composante EA1 ;
- l'intervalle de temps (*LargEA1*) séparant i) ledit franchissement du seuil d'amplitude ou d'énergie de la composante EA1 de ii) l'instant du pic de la composante EA1 ; et
- un paramètre composite combinant des paramètres précédents,
la composante EA1 étant la composante correspondant au premier pic du signal EA associé à la contraction ventriculaire isovolumique, et la composante EA2 étant la composante correspondant au second pic du signal EA associé à la relaxation ventriculaire isovolumique.

5. Le dispositif de la revendication 1, comprenant en outre une horloge interne (14) et des moyens aptes à restreindre la détermination de la période de sommeil à une plage horaire quotidienne prédéterminée.

6. Le dispositif de la revendication 1, comprenant en outre des moyens (16) de détection de phases d'activité du patient, et des moyens aptes à exclure du calcul de l'indice de variabilité les valeurs du paramètre EA mémorisées pendant des phases d'activité détectées au cours de la période de sommeil.

7. Le dispositif de la revendication 1, comprenant en outre des moyens (18) de détection de phases de réveil du patient, et des moyens aptes à exclure du calcul de l'indice de variabilité les valeurs du paramètre EA mémorisées pendant des phases de réveil détectées au cours de la période de sommeil.

8. Le dispositif de la revendication 1, comprenant en outre un compteur (c) de franchissements par ledit ratio dudit seuil d'alerte prédéterminé, et dans lequel les moyens de diagnostic sont aptes à délivrer le signal d'alerte lorsque le compteur atteint une valeur cumulée prédéterminée.

9. Le dispositif de la revendication 8, dans lequel le compteur n'est incrémenté qu'en cas de franchissements consécutifs dudit seuil d'alerte prédéterminé.

10. Le dispositif de la revendication 1, dans lequel ladite valeur d'indice de variabilité de référence est une valeur fixe prédéterminée.

11. Le dispositif de la revendication 1, dans lequel ladite valeur d'indice de variabilité de référence est une valeur (*VPEA1₁*) de l'indice de variabilité initialement calculée, puis mémorisée par les moyens d'évaluation.

12. Le dispositif de la revendication 11, comprenant des moyens aptes, lorsque ledit ratio franchit un seuil prédéterminé d'amélioration d'état du patient inférieur audit seuil d'alerte prédéterminé, à mettre à jour, par la valeur d'indice de variabilité courante calculée, ladite valeur d'indice de variabilité de référence mémorisée par les moyens d'évaluation.

## Patentansprüche

1. Aktive medizinische Vorrichtung, insbesondere eine implantierbare Stimulations-, Resynchronisations- und/oder Defibrillationsvorrichtung, oder ein Gerät zu Diagnosezwecken, die aufweist:
- einen Sensor (12), der ein Signal der endokardialen Beschleunigung EA liefern kann;
- Einrichtungen (10) zur Signalanalyse, die aus dem Signal EA einen vorbestimmten Parameter EA (*PEA1*) entnehmen und diesen Parameter EA über die aufeinanderfolgenden Herzzyklen speichern können;
- Einrichtungen (10) zur Bestimmung einer Schlafperiode (n) des Patienten; und
- Einrichtungen (20, 22) zur Bewertung des hämodynamischen Zustands des Patienten, die einen klinischen Zustandsindex des die Vorrichtung tragenden Patienten abhängig von Schwankungen des Parameters EA über die Schlafperiode (n) des Patienten liefern können,
**dadurch gekennzeichnet, dass** die Bewertungseinrichtungen (20, 22) Einrichtungen sind, die in der Lage sind:
· einen Variabilitätsindex (*VPEA1ₙ*) des Parameters EA über die Schlafperiode (n) zu berechnen,
· ein Verhältnis (R) zwischen dem berechneten Variabilitätsindex und einem Bezugsvariabilitätsindexwert *(VPEA1₁)* zu berechnen, und
· das Verhältnis als klinischen Zustandsindex zu liefern;
und dass die Vorrichtung außerdem Einrichtungen (26) zur Diagnose einer Herzinsuffizienz aufweist, die ein Alarmsignal betreffend die Verschlechterung des Zustands des Patienten als Reaktion auf die Überschreitung einer vorbestimmten Alarmschwelle durch das Verhältnis liefern können.

2. Vorrichtung nach Anspruch 1, wobei der Variabilitätsindex ausgehend von der Standardabweichung der Werte des Parameters EA über die Schlafperiode bestimmt wird.

3. Vorrichtung nach Anspruch 2, wobei der Variabilitätsindex die Standardabweichung der Werte des Parameters EA über die Schlafperiode ist, gewichtet durch den Mittelwert dieser Werte über die gleiche Periode.

4. Vorrichtung nach Anspruch 1, wobei der Parameter EA ein Parameter der Gruppe ist, die enthält:
- den Wert (*PEA1*) der Spitze-Spitze-Amplitude der Komponente EA1;
- den Wert (*PEA2*) der Spitze-Spitze-Amplitude der Komponente EA2;
- das Zeitintervall (*Syst*), das den Beginn der Komponente EA1 vom Beginn der Komponente EA2 trennt;
- die Zeit (*TstEA1*) des Auftretens der Komponente EA1, dargestellt durch das i) einen Zeitmarker des Beginns eines Herzzyklus und ii) die Überschreitung einer Amplituden- oder Energieschwelle der Komponente EA1 trennende Intervall;
- das Zeitintervall (*LargEA1*)*,* das i) die Überschreitung der Amplituden- oder Energieschwelle der Komponente EA1 vom ii) Zeitpunkt des Spitzenwerts der Komponente EA1 trennt; und
- einen Verbundparameter, der die vorhergehenden Parameter kombiniert,
wobei die Komponente EA1 die Komponente ist, die dem ersten Spitzenwert des Signals EA entspricht, der der isovolumetrischen ventrikulären Kontraktion zugeordnet ist, und die Komponente EA2 die Komponente ist, die dem zweiten Spitzenwert des Signals EA entspricht, der der isovolumetrischen ventrikulären Relaxation zugeordnet ist.

5. Vorrichtung nach Anspruch 1, die außerdem einen inneren Taktgeber (14) und Einrichtungen enthält, die die Bestimmung der Schlafperiode auf eine vorbestimmte tägliche Zeitspanne beschränken können.

6. Vorrichtung nach Anspruch 1, die außerdem Einrichtungen (16) zur Erfassung von Aktivitätsphasen des Patienten und Einrichtungen enthält, die aus der Berechnung des Variabilitätsindex die Werte des Parameters EA ausschließen können, die während im Laufe der Schlafperiode erfassten Aktivitätsphasen gespeichert wurden.

7. Vorrichtung nach Anspruch 1, die außerdem Einrichtungen (18) zur Erfassung von Wachphasen des Patienten und Einrichtungen enthält, die aus der Berechnung des Variabilitätsindex die Werte des Parameters EA ausschließen können, die während im Laufe der Schlafperiode erfassten Wachphasen gespeichert wurden.

8. Vorrichtung nach Anspruch 1, die außerdem einen Zähler (c) von Überschreitungen der vorbestimmten Alarmschwelle durch das Verhältnis enthält, und wobei die Diagnoseeinrichtungen in der Lage sind, das Alarmsignal zu liefern, wenn der Zähler einen vorbestimmten kumulierten Wert erreicht.

9. Vorrichtung nach Anspruch 8, wobei der Zähler nur im Fall von aufeinanderfolgenden Überschreitungen der vorbestimmten Alarmschwelle inkrementiert wird.

10. Vorrichtung nach Anspruch 1, wobei der Bezugsvariabilitätsindexwert ein vorbestimmter Festwert ist.

11. Vorrichtung nach Anspruch 1, wobei Bezugsvariabilitätsindexwert ein Wert (*VPEA1₁*) des Variabilitätsindex ist, der anfangs berechnet, dann durch die Bewertungseinrichtungen gespeichert wird.

12. Vorrichtung nach Anspruch 11, die Einrichtungen enthält, die, wenn das Verhältnis eine vorbestimmte Schwelle der Zustandsverbesserung des Patienten überschreitet, die niedriger ist als die vorbestimmte Alarmschwelle, durch den berechneten laufenden Variabilitätsindexwert den von den Bewertungseinrichtungen gespeicherten Bezugsvariabilitätsindexwert aktualisieren können.

## Claims

1. An active medical device, in particular an implantable device for stimulation, resynchronisation and/or defribrillation, or a device for diagnosis purpose, including:
- a sensor (12) adapted to deliver an endocardial acceleration, EA, signal;
- signal analysis means (10), adapted to extract from the EA signal a predetermined EA parameter (*PEA1*) and to memorize this EA parameter over the successive heart cycles;
- means (10) for determining a sleep period (n) of the patient; and
- means (20, 22) for evaluating the patient's hemodynamic state, adapted to deliver an index of clinical state of the patient wearing the device as a function of the variations of the EA parameter over said patient's sleep period (n),
**characterized in that** the evaluation means (20, 22) are means adapted to:
. calculate an index of variability (*VPEA1ₙ*) of said EA parameter over the sleep period (n),
. calculate a ratio (R) between said calculated index of variability and a reference value of index of variability (*VPEA1₁*), and
. deliver said ratio as a clinical state index;
and **in that** the device further includes heart failure diagnosis means (26), adapted to deliver a patient's state aggravation alert signal in response to said ratio crossing a predetermined alert threshold.

2. The device of claim 1, wherein said index of variability is determined from the standard deviation of the values of the EA parameter over the sleep period.

3. The device of claim 2, wherein said index of variability is the standard deviation of the values of the EA parameter over the sleep period, weighted by the average of these values over the same period.

4. The device of claim 1, wherein said EA parameter is a parameter of the group comprising:
- the value (*PEA1*) of the peak-to-peak amplitude of the component EA1;
- the value (*PEA2*) of the peak-to-peak amplitude of the component EA2;
- the time interval (*Syst*) separating the beginning of the component EA1 from the beginning of the component EA2;
- the time (*TstEA1*) of appearance of the component EA1, represented by the interval separating i) a time marker of beginning of hear cycle and ii) the crossing of a threshold of amplitude or energy of the component EA1;
- the time interval (*LargEA1*) separating i) said crossing of the threshold of amplitude or energy of the component EA1 from ii) the instant of the component EA1 peak; and
- a composite parameter combining previous parameters,
the component EA1 being the component corresponding to the first peak of the EA signal associated with the isovolumic ventricular contraction, and the component EA2 being the component corresponding to the second peak of the EA signal associated with the isovolumic ventricular relaxation.

5. The device of claim 1, further comprising an internal clock (14) and means adapted to restrict the determination of the sleep period to a predetermined daily time slot.

6. The device of claim 1, further comprising means (16) for detecting patient's activity phases, and means adapted to exclude from the calculation of the index of variability the EA parameter values memorized during activity phases detected during the sleep period.

7. The device of claim 1, further comprising means (18) for detecting patient's wake-up phases, and means adapted to exclude from the calculation of the index of variability the values of the EA parameter memorized during wake-up phases detected during the sleep period.

8. The device of claim 1, further comprising a counter (c) for counting the crossings by said ratio of said predetermined alert threshold, and wherein the diagnosis means are adapted to deliver the alert signal when the counter reaches a predetermined cumulated value.

9. The device of claim 8, wherein the counter is incremented only in case of consecutive crossings of said predetermined alert threshold.

10. The device of claim 1, wherein said reference value of index of variability is a predetermined fixed value.

11. The device of claim 1, wherein said reference value of index of variability is a value (*VPEA1₁*) of the index of variability initially computed, then memorized by the evaluation means.

12. The device of claim 11, comprising means adapted, when said radio crosses a predetermined patient's state improvement threshold lower than said predetermined alert threshold, to update, by the computed current value of index of variability, said reference value of index of variability memorized by the evaluation means.
